# EUROPÄISCHE PATENTSCHRIFT

(11) **EP 2 114 274 B1**
(45) Veröffentlichungstag und Bekanntmachung des Hinweises auf die Patenterteilung: **15.04.2015**
(21) Anmeldenummer: 08706779.9
(22) Anmeldetag: 22.01.2008
(51) Int. Cl.: A61B 17/70, A61B 17/80

(54) **PLATTENIMPLANTAT, INSBESONDERE FÜR DIE ANWENDUNG AN EINER WIRBELSÄULE**
PLATE-IMPLANT, IN PARTICULAR FOR USE ON A SPINAL COLUMN
IMPLANT SOUS FORME DE PLAQUE, À UTILISATION DESTINÉE NOTAMMENT À LA COLONNE VERTÉBRALE

(30) Priorität: 30.01.2007 DE 202007001585 U
(43) Veröffentlichungstag der Anmeldung: 11.11.2009
(73) Patentinhaber: Ulrich GmbH & Co. KG, 89081 Ulm (DE)
(72) Erfinder: REITZIG, Cliff-Georg, 78604 Rietheim-Weilheim (DE); ECKHOF, Stephan, 78604 Rietheim-Weilheim (DE); SCHWEIZER, Barbara, 78573 Wurmlingen (DE)
(74) Vertreter: Hentrich Patentanwälte PartG mbB
(86) Internationale Anmeldenummer: PCT/DE2008/000100
(87) Internationale Veröffentlichungsnummer: WO 2008/092422

(56) Entgegenhaltungen:
- WO-A-99/04718
- WO-A-02/098276
- US-A1- 2004 215 195

## Beschreibung

### Technisches Gebiet

Die Erfindung bezieht sich auf ein Plattenimplantat, das in seiner Grundausstattung aus einer ersten und einer zweiten Plattenkomponente besteht. Die erste Plattenkomponente weist Bohrungen zur Aufnahme von Befestigungsschrauben und einem Verbindungsmittel auf. Die weitere zweite Plattenkomponente zeigt ein Aufnahmemittel zur Aufnahme eines Verbindungsmittels und Bohrungen zur Aufnahme von Befestigungsschrauben. Jede der Plattenkomponenten ist bezüglich der anderen Plattenkomponente in eine Richtung gleitend gelagert und die Plattenkomponenten sind mit einer Vorrichtung versehen, die ihren Gleitlauf zueinander begrenzen.

### Stand der Technik

Die Osteosynthese ist ein Verfahren, das zur Versorgung von Knochenbrüchen und anderen Knochenverletzungen dient, bei denen insbesondere Implantate, die meistens aus Metall bestehen, eingesetzt werden. Ziel ist es dabei, die Fixierung der zueinander gehörigen Knochenfragmente in einer normalen Stellung (Reposition) zu fixieren. In der Regel erfolgt die operative Versorgung durch das Anbringen von Metallplatten und Befestigungsschrauben am Knochen oder, besonders bei Frakturen des Schaftes der grossen Röhrenknochen, durch Einbringung von langen Nägeln, die in der Markhöhle entlang der Achse des Knochens gelagert werden.

Insbesondere sind bei der Knochenzusammenführung im Bereich der Halswirbelsäule spezielle Techniken bekannt. Sie werden üblicherweise bei Krankheitsbildern, die auf ein HWS (Halswirbelsäulen) - Syndrom zurückzuführen sind, aber auch bei einem degenerativen Rückgrat eingesetzt. Die bei HWS-Syndromen angewandten Techniken stützen sich darauf, dass operativ die so genannte craniocervikale Instabilität durch Einfügen von Plattenimplantaten verringert beziehungsweise beseitigt wird. Hierzu werden plattenartige Elemente, die mehrere Bohrungen zur Aufnahme von Befestigungsschrauben umfassen, auf die dem Körper abgewandten Seite der Wirbelsäule angebracht, sodass mit den Befestigungsschrauben mehrere aufeinander folgende Wirbelkörper miteinander verbunden werden.

Insbesondere stellt sich bei der Befestigung des Plattenimplantats die Schwierigkeit, dass in der Regel der Abstand zwischen den aufeinanderfolgenden Wirbelkörpern oft unterschiedlich ist, sodass eine Vielzahl von unterschiedlichen langen Plattenimplantaten in unterschiedlichen Ausgestaltungen bevorratet werden muss.

Um diesen Umstand zu vermeiden, ist beispielsweise aus der US 5616142 A (YUAN HANSEN A (US); LIN CHIH-I (US)) 01.04.1997 ein Plattenimplantat bekannt, das aus zwei Plattenkomponenten besteht, die jeweils Aufnahmemittel zur Aufnahme von Verbindungsmitteln einer Zwischenplatte aufweisen. Die Verbindungsmittel selbst sind in den Aufnahmemitteln gleitend gelagert, sodass der Abstand von den beiden Plattenkomponenten zueinander variabel gestaltet werden kann. Die Plattenkomponenten selbst weisen Bohrungen auf, in die die Befestigungsschrauben einbringbar sind. Sobald der Abstand der beiden Plattenkomponenten zueinander fixiert ist, wird die Zwischenplatte an den Plattenkomponenten fixiert, sodass ein steifes, nicht mehr zu verschiebendes Plattenimplantat entsteht. Damit ist die Eigenschaft einer einfachen Anpassung an verschiedene Zwischenräume der Knochen beziehungsweise Wirbelkörper gegeben.

Aus der WO 99/004718 (DIMSO DISTRIBUTION MEDICALE DU SUD OUEST) 04.02.1999 ist ein Plattenimplantat, insbesondere eine Knochenplatte für die Anwendung an der Vorderseite einer Wirbelsäule bekannt. Dieses Knochenimplantat zielt darauf ab, insbesondere eine Anwendung an den vorderen Halswirbeln zu bieten. Es besteht aus zwei zueinander gleitenden Plattenelementen, die jeweils Bohrungen zur Aufnahme von Befestigungsschrauben aufweisen. Ferner weisen beide ein Verbindungsmittel beziehungsweise ein Aufnahmemittel auf, sodass beide Plattenelemente zueinander gleitend beweglich sind. Aufgrund der Gleitbewegung kann der Abstand der Befestigungsschrauben der jeweiligen Plattenelemente ausgewählt werden, sodass die Befestigungsschrauben in dem Knochen ausreichend Halt finden. Ist die Position entsprechend gewählt, können die beiden Knochen beziehungsweise Wirbelkörper zusammengedrückt werden, wodurch eine entsprechende Gleitbewegung der beiden Plattenimplantate entsteht. Ist die gewünschte Position erreicht, kann durch Einsetzen einer Fixierschraube die Gleitbewegung unterbrochen und damit vollständig blockiert werden, sodass ein form- und kraftschlüssiges Implantat entsteht.

Aus der US 2002/0183755 A1 ist ein Plattenimplantat mit den Merkmalen des Oberbegriffs des Hauptanspruchs bekannt, das eine Dynamisierung ermöglicht derart, dass eine Verkürzung, und nur eine Verkürzung, ermöglicht, indem die Oberfläche der Plattenkomponenten ein Sägezahnelement aufweisen.

### Nachteile der Standes der Technik

Unter dem Begriff "Settling" oder "Sinking" (nachstehend stellvertretend für Settling genannt) ist eine Migration der Knochen beziehungsweise Wirbelkörper zu verstehen, die aufgrund der Nichtbelastung (wegen des Anbringens des Implantats) degenerieren und ihre Lage entsprechend verändern. Bevor ein Plattenimplantat angebracht wird, wird zwischen die Wirbelkörper ein Platzhalter eingelegt. Platzhalter können aus einem körperfremden oder einem körpereigenen (beispielsweise Knochen) Material bestehen. Dieser Platzhalter fixiert sich aufgrund der Kräfte, die auf die Wirbelkörper wirken. Doch wegen dauerhafter axialer und vertikaler Belastung degenerieren die Wirbelkörper und der jeweilige Platzhalter verlässt seine vorgegebene Position. Sinkt der Platzhalter in den Wirbelkörper (aufgrund starker Belastung, Abnutzung, schlechter Qualität des Knochens etc.), so entsteht zwischen den Wirbelkörpern ein Freiraum, da die Wirbelkörper sich nicht mehr aufeinander zu bewegen können, da ihr Abstand zueinander durch die aus dem Stand der Technik bekannten Plattenimplantate fixiert ist. Die axialen und vertikalen Belastungen werden nun nicht mehr über die Wirbelkörper und den Platzhalter geführt, sondern der Kraftfluss wird über das Plattenimplantat geführt.

Die dabei entstehenden Kräfte aufgrund dieses Settlings- beziehungsweise Sinking-Vorgangs sind derart groß, dass die Befestigungsschrauben, die an sich zur Fixierung der Plattenimplantate vorgesehen sind, sich lösen. Dadurch ist das Implantat nicht mehr funktionsfähig und es können weitere Schäden an Knochen und Wirbelkörpern entstehen. Es besteht auch die Gefahr, dass aufgrund der hohen auftretenden Kräfte das Plattenimplantat bricht. Damit kann die eigentliche Funktion des Plattenimplantats vollständig außer Kraft gesetzt werden.

Somit besteht einer der wesentlichen Nachteile der aus dem Stand der Technik bekannten Plattenimplantate darin, dass das sogenannte Settling von den Implantaten nicht berücksichtigt wird.

### Aufgabe der Erfindung

Die Aufgabe der Erfindung ist es, Plattenimplantate gemäß dem Stand der Technik derart weiter zu entwickeln, dass insbesondere das Settling von Knochen beziehungsweise Wirbelkörpern, wenn diese mit einem Plattenimplantat versehen sind, ausgleicht.

### Lösung der Erfindung

Die vorliegende Erfindung zielt darauf ab, die Nachteile des Standes der Technik durch ein Plattenimplantat nach Anspruch 1 zu vermeiden.

### Vorteile der Erfindung

Die Erfindung geht einen vollständig anderen Weg, als den, den der Stand der Technik vorschlägt. Aufgrund der aufgeführten Klemmwirkung wird im Gegensatz zum Stand der Technik, bei dem eine vollständige Blockade der Gleitbewegung der Plattenkomponenten nach der Justierung erzielt wird, erreicht, dass durch das Settling eine Verschiebung der beiden Plattenkomponenten zueinander entgegen einer definierten Klemmkraft möglich ist. Die Klemmkraft wird durch da Anzugsmoment der Klemmschraube, aber auch durch die Klemmwirkung, die der Schraubenkopf der Klemmschraube durch Zusammenwirken mit der Längsbohrung erzielt, hervorgerufen.

Damit können sich die Knochen bzw. Wirbelkörper aufeinander zubewegen, wenn der Platzhalter nicht mehr den an sich gedachten Platz aufzeigt. Dadurch verbleibt der Kraftfluss zwischen den Knochen beziehungsweise Wirbelkörpern und verläuft nicht über das Plattenimplantat selbst, wodurch gewährleistet ist, dass die Befestigungsschrauben nicht mit derart hohen Kräften belastet werden, dass eine Lockerung oder sogar eine Loslösung aus dem Knochen beziehungsweise aus den Wirbelkörpern erfolgen kann.

Ein weiterer Vorteil der Erfindung ist es, dass das Plattenimplantat hinsichtlich seiner Führungselemente derart ausgestaltet ist, dass bei Verschiebungen des Plattenimplantats Teile dieses Implantats nicht über den Wirbelkörper bzw. Knochen gleiten. Dadurch werden Irritationen der Knochenhaut vorteilhafterweise vermieden.

Einer der weiteren Vorteile der Erfindung besteht darin, dass das Plattenimplantat aus beliebigem Material herstellbar ist. So ist es zum Beispiel denkbar, dass es neben Titan auch aus spritzgussfähigem Kunststoff herstellbar ist.

Die Geometrie der Plattenkomponenten ist sehr klein gewählt. Dies bringt den Vorteil mit sich, dass diese einzelnen Plattenkomponenten exakt auf die jeweiligen Wirbelkörper aufgesetzt werden können, ohne dass diese einen Überstand (über die Geometrie des Wirbelkörpers hinaus) bilden. Denn aus dem Stand der Technik ist bekannt, dass insbesondere zu lang gewählte Plattenelemente, die über in den Plattenelementen vorgesehene Langlöcher mit Schrauben befestigt werden, sich beim Settlingvorgang in den benachbarten Wirbelkörper oder in die Bandscheiben schieben und so Sekundärschäden hervorrufen.

Vorzugsweise sind Plattenbreiten bis kleiner 22 mm und Plattendicken kleiner als 2,5 mm vorgesehen.

Zudem bietet das Plattenimplantat der vorliegenden Erfindung gegenüber dem Stand der Technik den Vorteil, dass es vor der Adaption auf Knochen oder Wirbelkörper bereits zusammengefügt werden kann und Fixiermittel umfasst, mittels denen eine einfache und vorläufige Fixierung auf Knochen und Wirbelkörpern möglich ist, um so eine Anpassung der einzelnen Abstände und das Finden der optimalen Anbringung der Befestigungsschrauben auf einfache Art und Weise zu ermöglichen.

Ein weiterer Vorteil der Erfindung besteht darin, dass das Plattenimplantat als Bausatz vorliegt. Unter Zugrundelegung der jeweiligen Plattenkomponenten kann durch Einfügen unterschiedlicher Anzahl von Erweiterungsplatten die Länge des Plattenimplantats je nach Anwendungsfall frei gewählt werden. Dabei bleibt weiterhin die eingeschränkte Gleitbewegung zwischen jeder einzelnen Platte gewährleistet. Die einzelnen Plattenkomponenten und die Erweiterungsplatten weisen trotzdem eine sehr kleine Geometrie auf, so dass die zuvor genannten Sekundärschäden vermieden werden.

Die Erweiterungsplatten bestehen vorzugsweise ebenfalls aus einem plattenartigen Grundkörper, der Bohrungen für die Aufnahme von Befestigungsschrauben aufweist. Ferner sind Aufnahmemittel zur Aufnahme eines Verbindungsmittels und ein Verbindungsmittel selbst vorgesehen. Längsbohrungen zur Aufnahme einer weiteren Klemmschraube und zur Begrenzung des Gleitlaufs sind ebenfalls vorgesehen. Damit kann aus mindestens drei unterschiedlichen Plattenkomponenten ein Plattenimplantat definierter Länge hergestellt werden. Der Anwender kann unmittelbar vor Ort entscheiden, welche Länge notwendig ist. Diese kann durch die Anzahl der Erweiterungsplatten bestimmt werden. Dadurch entfällt eine erheblich kostenaufwendige Bevorratung von unterschiedlichen Plattenimplantaten unterschiedlicher Länge.

Die Plattenimplantate selbst weisen sowohl in ihrer Längs- als auch in ihrer Querstreckung Krümmungen auf. Zusätzlich sind sie aufgrund ihrer Dicke entsprechend an die Kontur der Knochen bzw. der Wirbelkörper anpassbar, indem sie von dem Anwender entsprechend zurechtgebogen werden können.

Dabei geht aber die Eigenschaft nicht verloren, dass die Verbindungsmittel in den Aufnahmemitteln frei gleiten können, damit das entsprechende Settling nachvollzogen werden kann.

Aufgrund der Ausgestaltung der einzelnen Plattenkomponenten beziehungsweise der Erweiterungsplatten ist es für den Anwender möglich, auf sehr einfache Art und Weise die Reihenfolge zur Montage des Plattenimplantats einzuhalten, da es aufgrund der einfachen optischen Struktur von Plattenkomponenten und Erweiterungsplatten ohne weiteres selbsterklärend erfasst werden kann, welche Plattenelemente zusammenzufügen sind.

Um diesen Vorgang weiter zu unterstützen, sind die einzelnen Plattenelemente farbig ausgestaltet, sodass der Anwender unmittelbar die Reihenfolge von erster Plattenkomponente, mindestens einer Erweiterungsplatte sowie der weiteren Plattenkomponente erfassen und umsetzen kann. Durch ein einfaches Stecksystem können die einzelnen Plattenelemente bereits funktionell miteinander zusammengefügt werden, sodass einzelne Gliederelemente entstehen. Die Funktionsfähigkeit kann bereits beim ersten Einstecken geprüft werden.

Durch Vorfixieren der Klemmschraube (noch nicht vollständiges Aufbringen eines Anziehmoments auf die Klemmschraube) bleibt zwar die Gleitbewegung der Plattenelemente untereinander erhalten, ist jedoch in den entsprechenden Bewegungsfreiheiten bereits eingeschränkt. Die vorgesehene Klemmkraft, die zwischen den Plattenelementen wirken soll, wird erst durch ein weiteres Hineindrehen der Klemmschraube in das Verbindungsmittel bewirkt.

Zusätzliche Bohrungen in den jeweiligen Plattenelementen können dafür vorgesehen werden, auf den Knochen beziehungsweise Wirbelkörpern die Plattenelemente vorzufixieren, damit ein Anbohren der Knochen zur Durchführung der Befestigung des Befestigungsschrauben möglich ist.

Die Bohrungen in den einzelnen Plattenelementen sind derart gewählt, dass die Schrauben polyaxial einbringbar sind. Dies bedeutet, dass die Befestigungsschrauben nicht senkrecht zu den einzelnen Plattenelementen eingefügt werden müssen. Sie sind in jedem beliebigen Winkel in den Knochen beziehungsweise in den Wirbelkörper eindrehbar. Vorzugsweise sind Befestigungsschrauben vorgesehen, die selbstsichernd sind. Hierzu können beispielsweise spreizbare Schraubenköpfe vorgesehen sein, sodass ein Klemmen der Schraube mit dem Plattenelement unmittelbar nach dem Fixieren auf dem Knochen beziehungsweise auf den Wirbelkörpern möglich ist.

Weitere vorteilhafte Ausgestaltungen gehen aus der nachfolgenden Beschreibung, den Ansprüchen sowie den Zeichnungen hervor. Kurze Beschreibung der Abbildungen der Zeichnungen
Fig. 1 zeigt eine perspektivische Ansicht auf das Plattenimplantat, bestehend aus einer ersten Plattenkomponente und einer zweiten Plattenkomponente, wobei die beiden Plattenkomponenten nicht miteinander verbunden sind;
Fig. 2 zeigt eine perspektivische Ansicht auf die in Fig. 1 dargestellten Plattenkomponenten jedoch mit Darstellungen der Befestigungsschrauben und der erfindungsgemässen Klemmschraube;
Fig. 3 zeigt eine perspektivische Ansicht auf ein Plattenimplantat, bestehend aus zwei Plattenkomponenten mit noch nicht fixierter Klemmschraube;
Fig. 4 zeigt eine perspektivische Ansicht auf das montierte Plattenimplantat gemäss Fig. 3, jedoch mit montierter Klemmschraube;
Fig. 5 zeigt eine Draufsicht auf ein montiertes Plattenimplantat bestehend aus zwei Plattenkomponenten im Bereich der Halswirbelsäule im nicht-gesettleten Zustand;
Fig. 6 zeigt eine Draufsicht auf ein montiertes Plattenimplantat, bestehend aus zwei Plattenkomponenten im Bereich der Halswirbelsäule im gesettleten Zustand;
Fig. 7 zeigt drei perspektivische Ansichten auf ein Erweiterungselement;
Fig. 8 zeigt eine perspektivische Ansicht eines Ausführungsbeispiels eines Plattenimplantats bestehend aus zwei Plattenkomponenten sowie einer Erweiterungsplatte;
Fig. 9 zeigt eine perspektivische Darstellung der Plattenkomponenten mit der Erweiterungsplatte zusammen mit Befestigungsschrauben und Klemmschrauben;
Fig. 10 zeigt eine perspektivische Ansicht auf ein zusammengefügtes Plattenimplantat bestehend aus zwei Plattenkomponenten und einer Erweiterungsplatte mit noch nicht eingefügten Klemmschrauben;
Fig. 11 zeigt eine perspektivische Ansicht auf ein zusammengefügtes Plattenimplantat bestehend aus zwei Plattenkomponenten und einer Erweiterungsplatte mit eingefügten Klemmschrauben;
Fig. 12 zeigt eine Draufsicht auf ein montiertes Plattenimplantat bestehend aus zwei Entplatten und drei Erweiterungsplatten, montiert im Bereich eines Halswirbels im nicht gesettleten Zustand;
Fig. 13 zeigt eine andere perspektivische Ansicht auf ein montiertes Plattenimplantat, bestehend aus zwei Entplatten und drei Erweiterungsplatten, montiert im Bereich eines Halswirbels im nicht- gesettleten Zustand;
Fig. 14 zeigt eine perspektivische Ansicht auf die Klemmschraube;
Fig. 15 zeigt einen Schnitt durch ein Plattenimplantat im Bereich der Klemmschraube;
Fig. 16 zeigt eine vergrösserte Darstellung des Klemmbereichs gemäss Fig. 15
Fig. 17 zeigt eine weitere vergrösserte Darstellung des Klemmbereichs im Bereich der Längsbohrung.

### Wege zur Ausführung der Erfindung

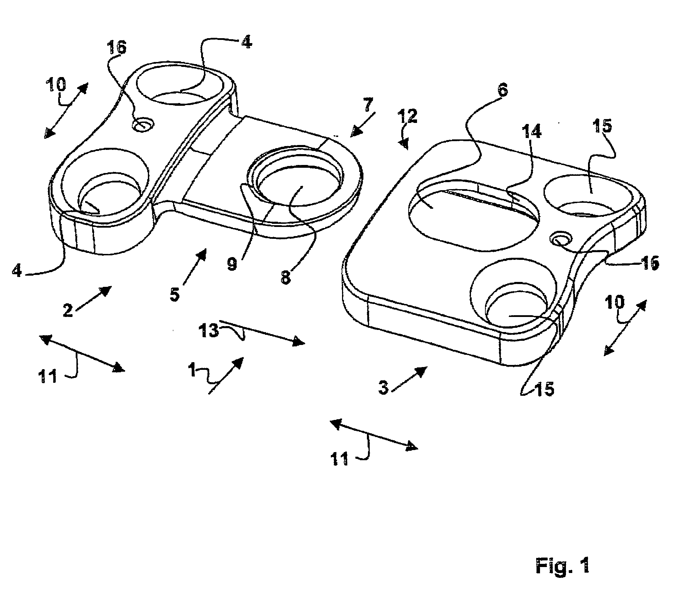

In Fig. 1 ist das Grundprinzip des Plattenimplantats 1 dargestellt. Dieses Plattenimplantat 1 besteht aus einer ersten Plattenkomponente 2 und einer weiteren Plattenkomponente 3. Die beiden Plattenkomponenten 2, 3 sind unterschiedlich gestaltet.

Die erste Plattenkomponente 2 weist Bohrungen 4 für noch nicht näher dargestellte Befestigungsschrauben auf. Ferner ist ein Verbindungsmittel 5 vorgesehen, das mit einem Aufnahmemittel 6 der weiteren zweiten Plattenkomponente 3 zusammenwirkt. Das Verbindungsmittel 5 ist zungenartig ausgestaltet und ist in seinem Querschnitt geringer ausgebildet als der übrige Teil der Plattenkomponente 2. Auch die Dicke des Verbindungsmittels 5 ist geringer als der übrige Teil der Plattenkomponente 1.

Im Bereich des freien Endes 7 des Verbindungsmittels 5 ist eine Bohrung 8 vorgesehen. Vorzugsweise weist diese Bohrung 8 auch ein Innengewinde 9 auf.

Die erste Plattenkomponente 2 ist sowohl in Richtung der Quererstreckung 10 als auch in Richtung der Längserstreckung 11 gebogen, sodass eine Anpassung an Wirbelkörper oder an Knochen erfolgen kann.

Wie bereits ausgeführt, ist das zungenartig ausgebildete Verbindungsmittel 5 auf das Zusammenwirken mit den Aufnahmeteil 6 der zweiten Plattenkomponente 3 ausgestaltet. Hierzu weist die zweite Plattenkomponente 3 als Aufnahmemittel 6 innerhalb des Grundkörpers der zweiten Plattenkomponente 3 eine Aussparung 12 auf, die derart ausgebildet ist, dass das zungenartige Verbindungsmittel 5 in Pfeilrichtung 13 in das Aufnahmemittel 6 eingeführt werden kann. Vorzugsweise ist zur exakten, nahezu spielfreien Führung eine Schwalbenschwanzführung vorgesehen. Das zungenartige Verbindungsmittel 5 ist derart ausgelegt, dass es auf der zum Wirbelkörper bzw. Knochen hinweisenden Seite nicht fluchtend mit der Unterseite der Plattenkomponenten 2, 3 ausgelegt ist. Es ist ein Abstand vorgesehen, um zu vermeiden, dass bei Gleitbewegungen dieses Verbindungsmittel 5 auf der Oberfläche des Knochens bzw. des Wirbelkörpers gleitet.

Die zweite Plattenkomponente 3 weist ferner eine Längsbohrung 14 auf, die im eingeschobenen Zustand der ersten Plattenkomponente 2 mit der Bohrung 8 des Verbindungsmittels 5 fluchtend ist.

Ferner weist die zweite Plattenkomponente 3 ebenfalls Bohrungen 15 zur Aufnahme von Befestigungsschrauben, die in der Fig. 1 so nicht dargestellt werden, auf. Jede der Plattenkomponenten 2, 3 weisen Bohrungen 16 auf, die in ihrem Durchmesser wesentlich geringer sind als die übrigen Bohrungen 4, 15. Diese Bohrungen 16 dienen dazu, um die Lage an dem Knochen oder den Wirbelkörpern der jeweiligen Plattenkomponenten 2, 3, bevor sie mit dem Befestigungsschrauben montiert werden, vorzufixieren.

Die zweite Plattenkomponente 3 ist in ihrer Form nahezu quadratisch ausgebildet und weist auch in ihrer Längserstreckung 11 als auch in der Quererstreckung 10 jeweils Krümmungsradien auf. Zusätzlich ist sie in diesen Richtungen ebenfalls biegbar, sodass sie an die Aussenkontur von Wirbelkörpern und Knochen anpassbar ist.

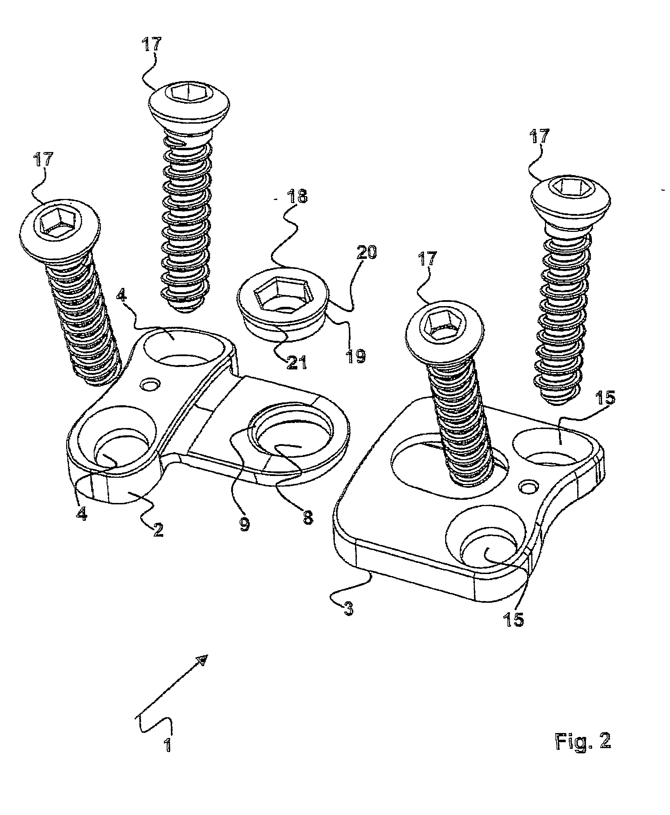

In Fig. 2 ist das Grundprinzip des Plattenimplantats 1 gemäss Fig. 1 dargestellt. Zusätzlich sind für die Bohrungen 4 beziehungsweise 15 Befestigungsschrauben 17 dargestellt, die polyaxial in die Bohrungen 4, 15 einführbar sind. Polyaxial bedeutet, dass diese nicht unmittelbar senkrecht zur Oberfläche der jeweiligen Plattenkomponente 2, 3 eingefügt werden müssen, sondern je nach Gegebenheit der Struktur, die sie antreffen.

Zusätzlich ist eine Klemmschraube 18 vorgesehen, die in das Gewinde 9 der Bohrung 8 eindrehbar ist. Die Klemmschraube 18 ist derart ausgebildet, dass sie ebenfalls ein Gewinde 19 aufweist, das mit dem Gewinde 9 der Bohrung 8 zusammenwirkt. Zusätzlich weist die Klemmschraube 18 im Bereich ihres Schraubenkopfes einen Wulst 20 auf, der vorzugsweise breiter gestaltet ist, als der übrige Teil des Gewindes 19. Dieser Wulst 20 weist eine nach Aussen hin verlaufende Schräge 21 auf, sodass der Durchmesser der Klemmschraube 18 von dem Gewinde 19 wegweisend grösser wird.

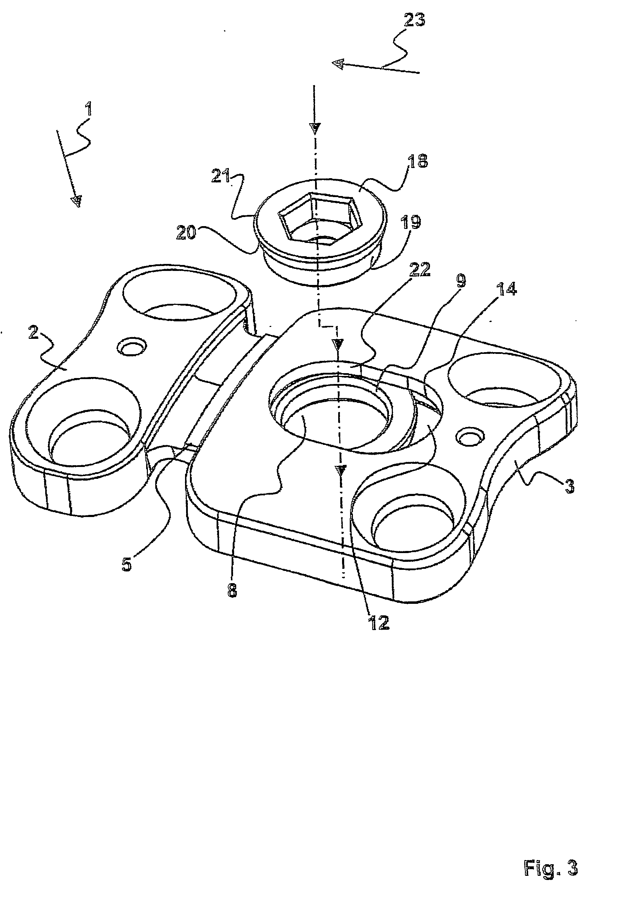

In Fig. 3 ist die Funktionsweise der Klemmschraube 18 in Verbindung mit dem Plattenimplantat 1 dargestellt. Sie wird bei zusammengefügten Plattenkomponenten 2, 3 in die Längsbohrung 14 der zweiten Plattenkomponente 3 eingefügt und das Gewinde 19 der Klemmschraube 18 tritt in Verbindung mit dem Gewinde 9 der Bohrung 8 des Verbindungsmittels 5. Der Wulst 20 tritt dabei mit den Seitenflanken 22 der Längsbohrung 14 in Verbindung derart, dass zwischen der Klemmschraube 18 und der Plattenkomponente 3 eine erste Klemmwirkung entsteht. Durch das Eindrehen der Klemmschraube 18 in die Bohrung 8 entsteht auch eine Klemmwirkung derart, dass das Verbindungsmittel 5 sich innerhalb der Aussparung 12 an die Innenseite der zweiten Plattenkomponente 3 drückt und hier eine Klemmwirkung entsteht. Es wird ausdrücklich darauf hingewiesen, dass die beiden Plattenkomponenten 2, 3 und die Klemmschraube 18 derart ausgebildet sind, dass kein Blockieren der Gleitbewegung in oder gegen die Pfeilrichtung 23 gewollt ist. Ziel ist es, eine Klemmwirkung zu erzielen, die ein Gleiten der ersten Plattenkomponenten 2 gegen die zweite Plattenkomponente 3 gegen eine definierte Kraft ermöglicht.

In der in Fig. 3 gezeigten Stellung wird die Klemmschraube 18 eingedreht, sodass die entsprechende Klemmwirkung erreicht wird. Wird durch ein so genanntes Settling eine Kraft auf das Plattenimplantat 1 ausgeübt, so bewegen sich die Plattenkomponenten 2, 3 aufeinander in Pfeilrichtung 23 zu. Die Kräfte müssen jedoch so gross sein, dass die hervorgerufene Klemmwirkung durch die Klemmschraube 18 überwunden wird. Erst dann findet eine Bewegung in Richtung Pfeilrichtung 23 statt.

Vorzugsweise bei einem anderen Ausführungsbeispiel kann auch die Längsbohrung 14 konisch ausgestaltet sein, sodass sie sich in Pfeilrichtung 23 verjüngt. Dies bedeutet, dass die Klemmkraft mit zunehmenden Settling grösser wird. Dies bedeutet auch, dass die Kräfte grösser sein müssen, um eine entsprechende Verschiebung in Pfeilrichtung 23 zu bewirken.

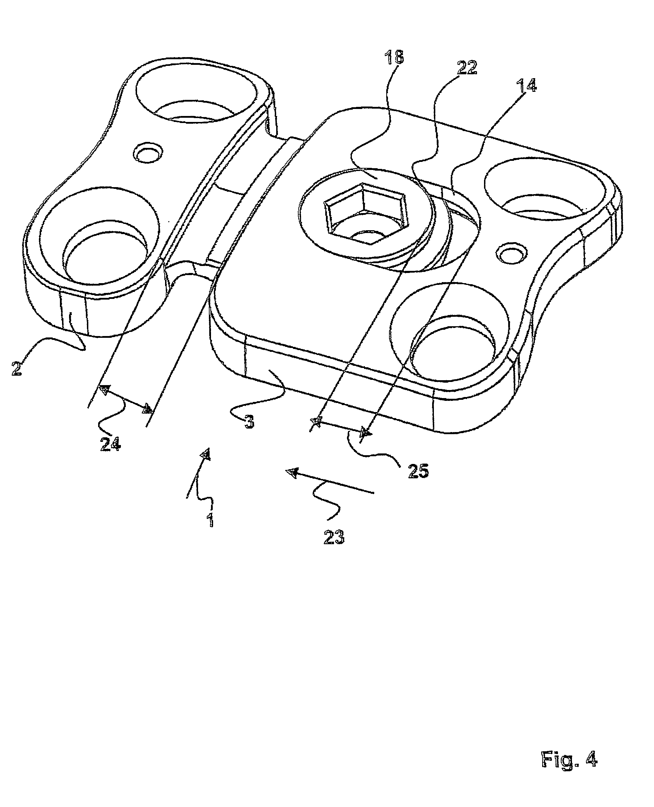

In Fig. 4 ist die in den vorherigen Fig. 1 und Fig. 2 dargestellte Grundform des Plattenimplantats 1 im montierten Zustand gezeigt. Die Klemmschraube 18 wirkt mit den Seitenflanken 22 der Längsbohrung 14 zusammen. Die Darstellung in Fig. 4 zeigt die Ausgangsstellung. Durch das Settling entsteht eine Verschiebung in Pfeilrichtung 23 und zwar die maximale Wegstrecke, die durch den Abstand 24 des einen Teils der ersten Plattenkomponente 2 zur zweiten Plattenkomponente 3 beziehungsweise durch den Abstand 25 begrenzt ist.

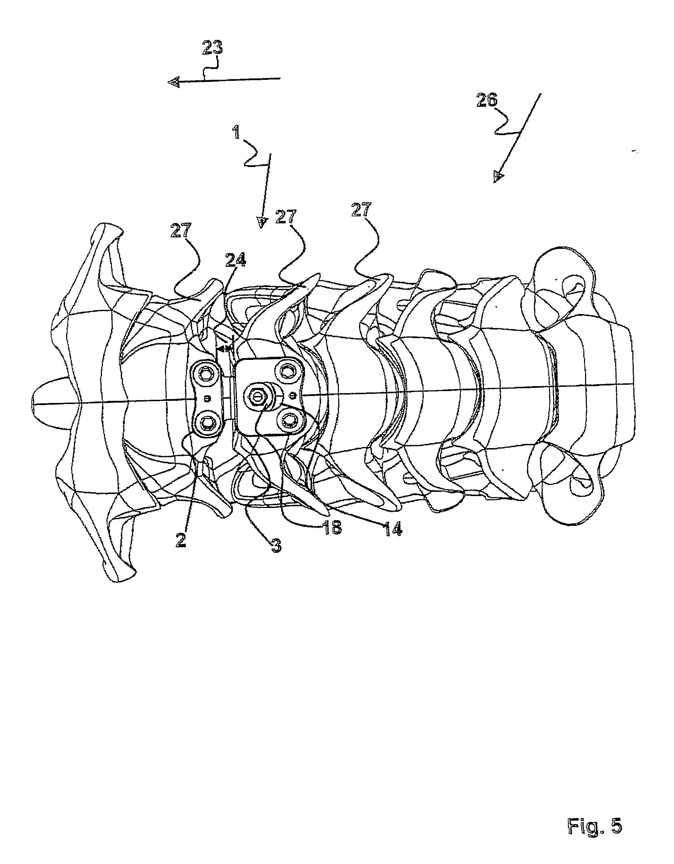

In Fig. 5 ist eine Draufsicht auf die Grundeinheit des Plattenimplantats 1 dargestellt. Die Draufsicht erfolgt auf einen Teil einer Wirbelsäule 26, die aus mehreren Wirbelkörpern 27 besteht. Bei dem hier dargestellten Ausführungsbeispiel ist die erste Plattenkomponente 2 ist an einem ersten Wirbelkörper 27 angeordnet, wobei die zweite Plattenkomponente 3 eine in dem nachfolgenden weiteren Wirbelkörper 27 angeordnet ist. Die beiden Plattenkomponenten 2, 3 weisen einen Abstand 24 auf, der frei gewählt worden ist, um ein Settling der beiden Wirbelkörper 27 zu kompensieren. Die Klemmschraube 18 ist derart innerhalb der Längsbohrung 14 angeordnet, dass sie auf der der ersten Plattenkomponente 2 zugewandten Seite angeordnet ist, damit eine Verschiebung der ersten Plattenkomponenten 2 gegenüber der zweiten Plattenkomponente 3 entgegen der Pfeilrichtung 23 beziehungsweise eine Verschiebung der zweiten Plattenkomponente 3 gegenüber der ersten Plattenkomponente 2 in Pfeilrichtung 23 möglich ist.

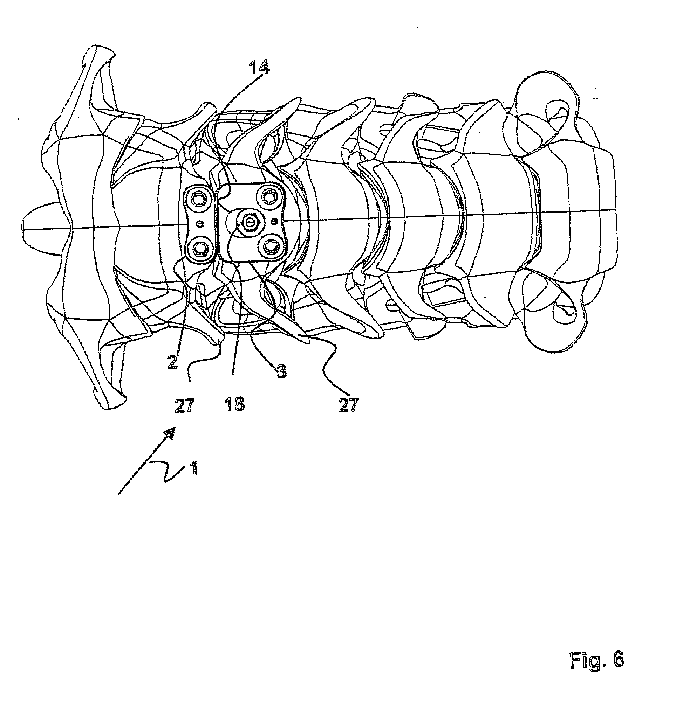

Wie in Fig. 6 dargestellt, ist ein Settling eingetreten und die beiden Plattenkomponenten 2, 3 haben sich aufeinander zubewegt. Die Klemmschraube 18 ist dabei innerhalb der Längsbohrung 14 geglitten und an ihrer Endposition angekommen. Die Wirbelkörper 27 liegen in dieser Position sehr eng beieinander, so dass weiterhin gewährleistet ist, dass der Kraftfluss nicht über das Plattenimplantat 1, sondern über die Wirbelkörper selbst erfolgt. Das Plattenimplantat 1 behält weiterhin seine Festigkeit gegenüber dem Wirbelkörper 27, da die Kräfte nicht auf die Befestigungsschrauben 17 wirken.

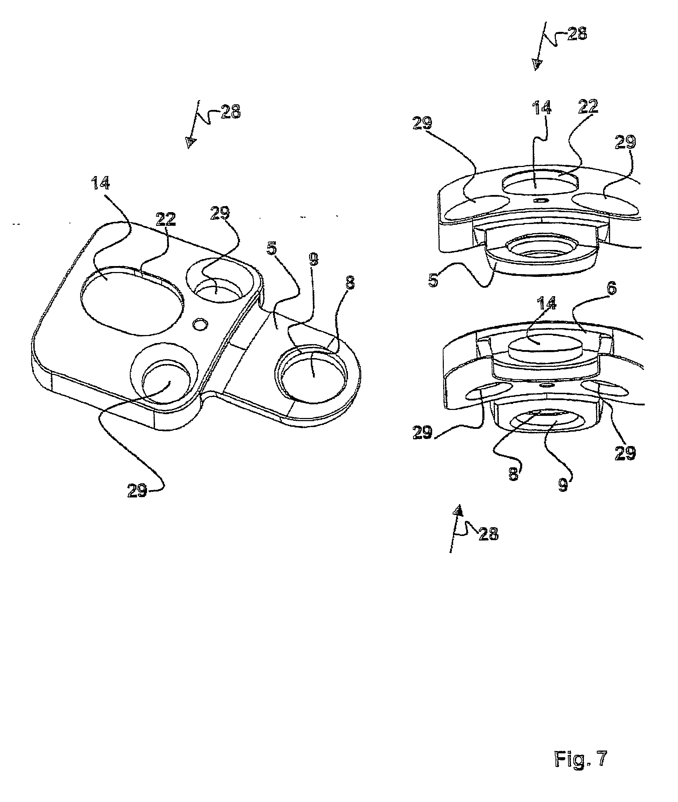

In Fig. 7 sind mehrere perspektivische Ansichten auf eine Erweiterungsplatte 28 dargestellt. Eine Erweiterungsplatte besteht ebenfalls wieder aus einem nahezu rechteckigen Grundkörper, an dessen einer Seite sich ein Verbindungsmittel 5 erstreckt. Dieses Verbindungsmittel 5 ist derart ausgestaltet, wie es bereits bei der ersten Plattenkomponente 2 gemäss Fig. 1 vorgesehen ist. Es ist zungenartig ausgebildet und weist an dessen Ende eine Bohrung 8 mit einem Innengewinde 9 auf. Ferner weist der Grundkörper 28 der Erweiterungsplatte zwei Bohrungen 29 auf, die dazu dienen, die in der Zeichnung nicht näher dargestellten Befestigungsschrauben aufzunehmen. Ferner ist im Grundkörper eine Längsbohrung 14 vorgesehen. Diese Längsbohrung entspricht der Längsbohrung 14, wie sie bereits aus der zweiten Plattenkomponente 3 bekannt ist. Sie hat ebenfalls eine längliche Aussparung und eine definierte Seitenflanke 22. Ferner weist die Erweiterungsplatte 28 ein Aufnahmemittel 6 auf. Dieses Aufnahmemittel 6 ist derart gestaltet, wie es bereits von der zweiten Plattenkomponente 3 bekannt ist. Es dient zur Aufnahme des Verbindungsmittels 5.

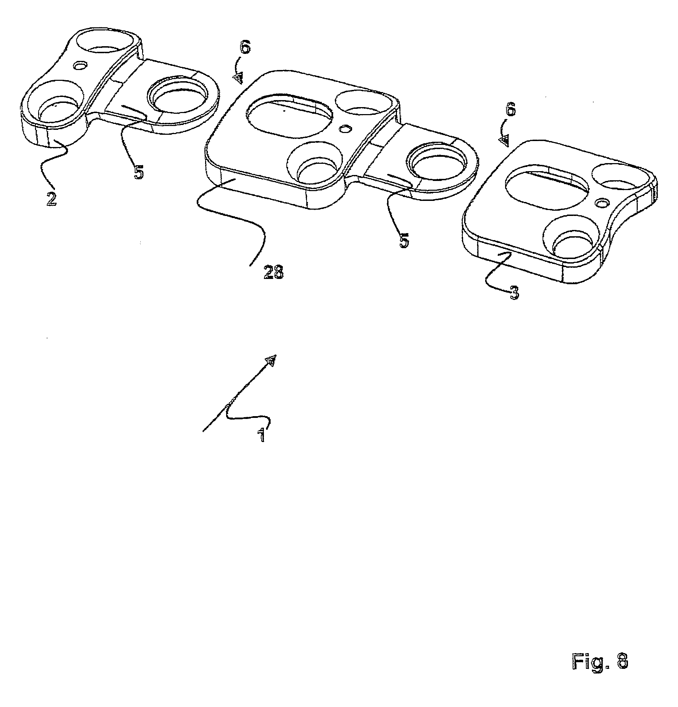

Fig. 8 zeigt in einer perspektivischen Ansicht die Anwendung einer solcher Erweiterungsplatte 28. Die Erweiterungsplatte 28 ist in der Ausbildung eines Kettengliedes zwischen die erste Plattenkomponente 2 und die zweite Plattenkomponente 3 einfügbar. Durch diese Konstellation wird ein weiteres mögliches Ausführungsbeispiel eines Plattenimplantats 1 gebildet. Je nach Bedarf können auch mehrere solcher Erweiterungsplatten 28 eingefügt werden, wobei immer gewährleistet sein muss, dass zu Beginn beziehungsweise zum Ende der "Kette" sowohl eine erste Plattenkomponente 2 als auch eine zweite Plattenkomponente 3 vorhanden sein muss. Diese beiden Plattenkomponenten 2, 3 bilden den Abschluss der "Kette".

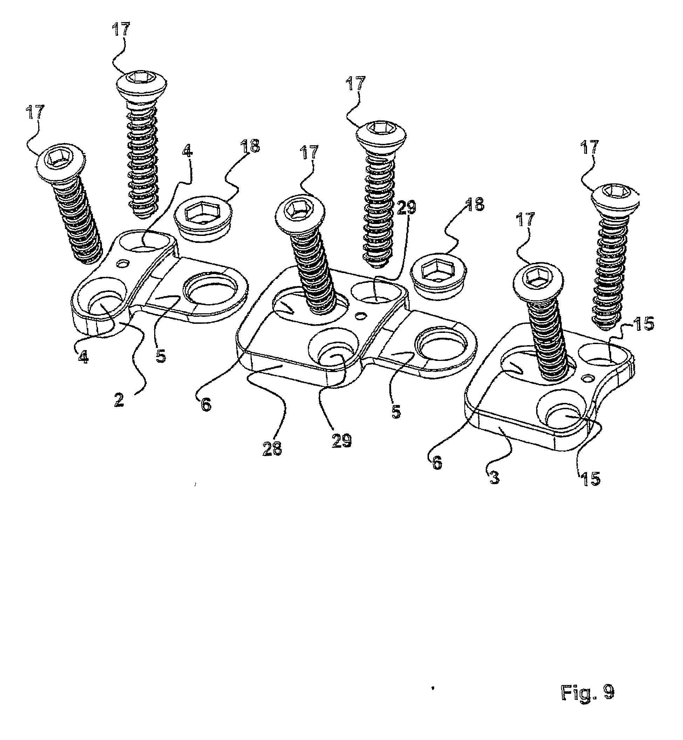

In Fig. 9 ist das gemäss Fig. 8 dargestellte Plattenimplantat 1 zusammen mit der Erweiterungsplatte 28 zusätzlich mit Befestigungsschrauben 17 und auch Klemmschrauben 18 dargestellt. Jedes der Plattenelemente weist mindestens zwei Bohrungen 4, 15, 29 auf, in die die Befestigungsschrauben 17 polyaxial einführbar sind. Durch Zusammenwirken der Klemmschrauben 18 mit den jeweiligen Verbindungselementen 5 und dem Aufnahmemittel 6 wird die entsprechende Klemmkraft bewirkt.

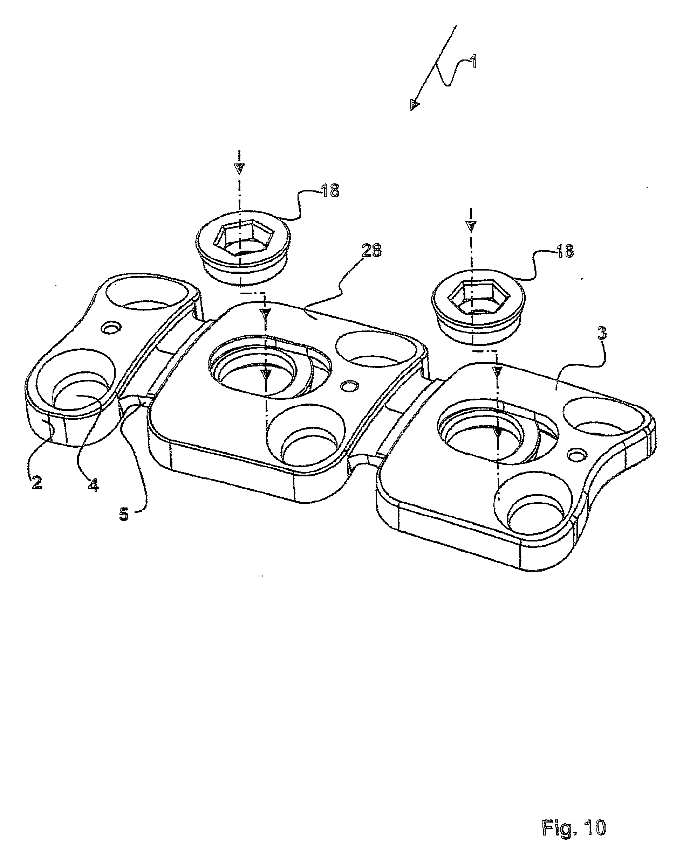

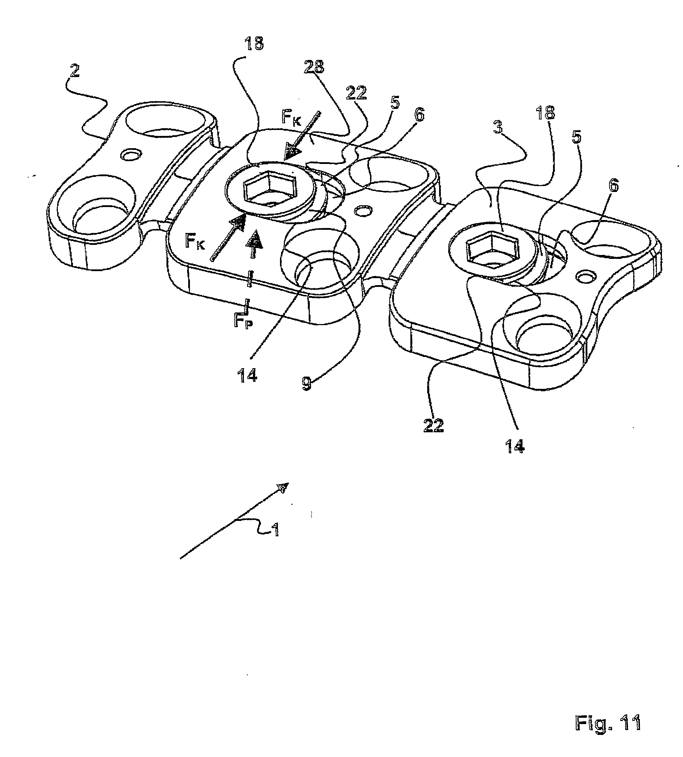

In den Fig. 10 und Fig. 11 ist die Montage und die Darstellung eines Ausführungsbeispiels eines Plattenimplantats 1, wie es in Fig. 8 und Fig. 9 bereits erläutert worden ist, dargestellt.

In der in Fig. 10 dargestellten Ansicht ist eine Gleitbewegung in uneingeschränkter Form wiedergegeben. Gegenüber Fig. 10 ist in Fig. 11 jeweils die Klemmschraube 18 eingedreht, derart, dass der Schraubenkopf der Klemmschraube 18 mit der Seitenflanke 22 der Längsbohrung 14 zusammenwirkt. Die dabei entstehenden Klemmkräfte F sind exemplarisch für alle Ausführungen der Zusammenwirkung der Klemmschraube 18 mit den jeweiligen Längsbohrungen 14 dargestellt. Zum einen wirken die Klemmkräfte F_{K}, die die Klemmschraube 18 gegenüber den Seitenflanken 22 der Längsbohrung 14 ausübt. Zum anderen wirken Klemmkräfte F_{P}, die durch das Einschrauben der Klemmschraube 18 in die Bohrung 8 des Verbindungsmittels 5 auftreten. Aufgrund dessen, dass sich der Schraubenkopf der Klemmschraube 18 an die Seitenflanken 22 der Längsbohrung anlegt, wird das Verbindungsmittel 5 an die Innenseite des Aufnahmemittels 6 herangezogen, sodass eine flächige Anlage erfolgt. Dadurch entsteht die weitere Klemmkraft F_{P}.

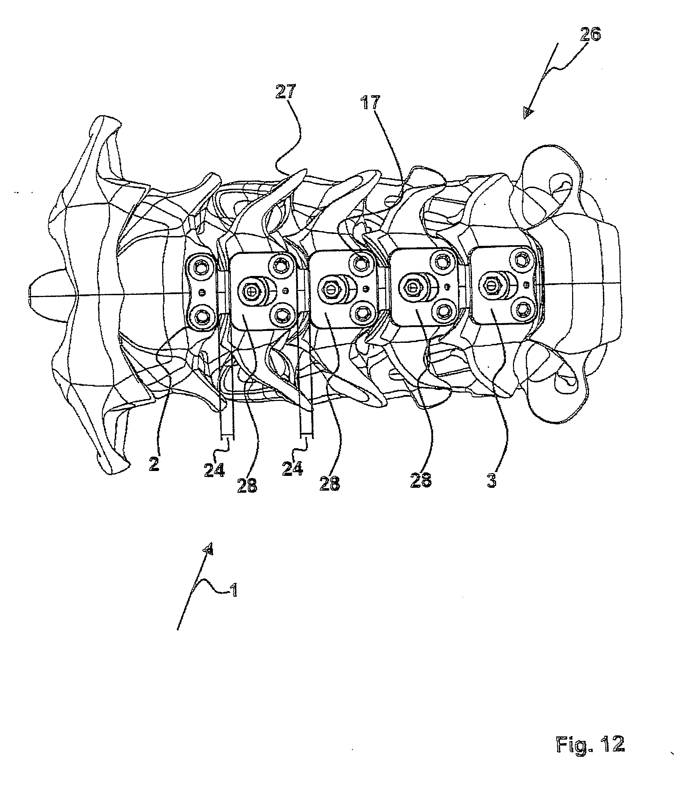

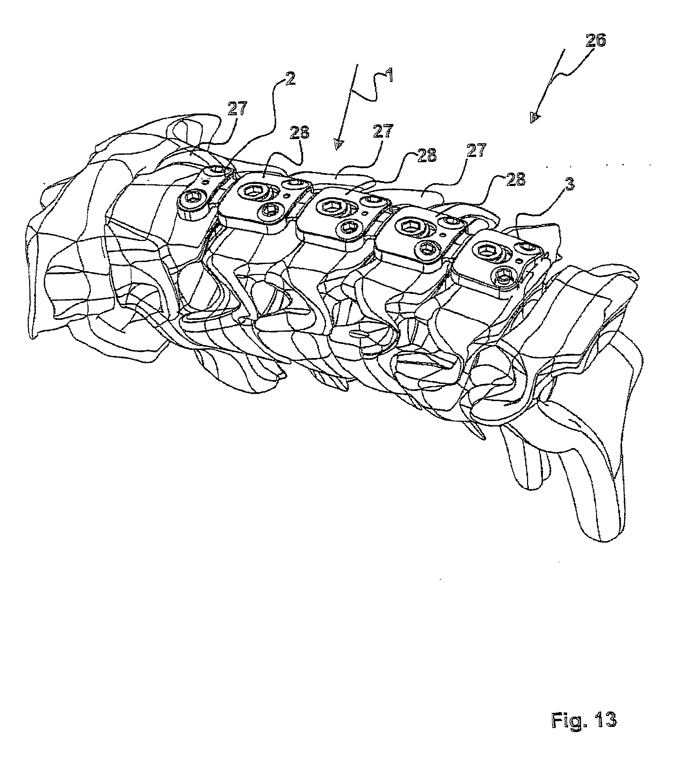

In Fig. 12 und Fig. 13 ist ein Anwendungsbeispiel dargestellt, das ein Plattenimplantat 1 zeigt, das aus der jeweiligen ersten Plattenkomponente 2 und der zweiten Plattenkomponente 3 sowie drei Erweiterungsplatten 28 besteht. Die Abstände der Erweiterungsplatten 28 sind derart gewählt, dass die Befestigungsschrauben 17 optimalen Halt in den Wirbelkörpern 27 der hier dargestellten Wirbelsäule 26 finden. Dabei ergibt sich zwischen den einzelnen Platten ein Abstand 24, der dafür benötigt wird, um das so zuvor erwähnte Settling auszugleichen.

Nachstehend wird die Funktionsweise der Klemmwirkung des Plattenimplantats 1 gezeigt. Insbesondere wird näher auf die Wirkung der Klemmschraube 18 im Zusammenwirken mit dem Verbindungsmittel 5 und dem Aufnahmemittel 6 eingegangen.

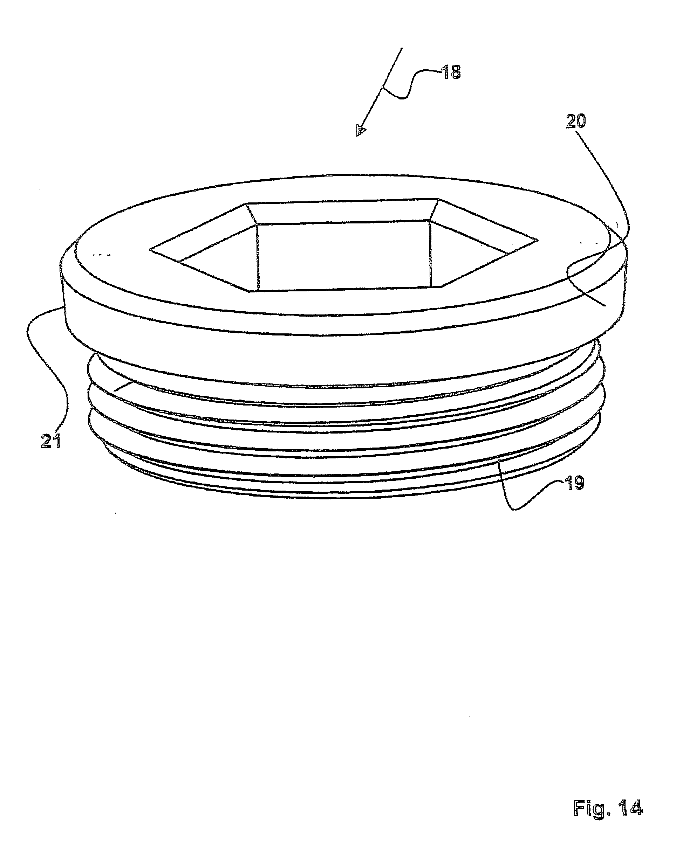

In Fig. 14 ist eine perspektivische Darstellung der Klemmschraube 18 gezeigt, wie sie in den vorherigen Figuren bereits eingesetzt worden ist. Die Klemmschraube weist im Wesentlichen zwei Bereiche auf, nämlich einen ersten Bereich eines Schraubenkopfes und einen weiteren Bereich, nämlich den Bereich eines Schraubengewindes. Der Bereich des Schraubengewindes umfasst ein Gewinde 19, das in der Regel handelsüblich ist. Es kann jedoch auch ein Feingewinde sein, damit bereits mit geringen Umdrehungen entsprechende Klemmkräfte erwirkt werden können.

Der Schraubenkopf ist speziell ausgestaltet. Er weist einen umlaufenden Wulst 20 auf, der in seinem Durchmesser zumindest gleich dem Schraubengewinde ist. Vorzugsweise ist der Schraubenkopf grösser als das Gewinde 19. Der Wulst 20 weist eine gewisse Dicke auf. Die Dicke ist derart ausgestaltet, dass sie umlaufend gleich ausgestaltet ist. Sie weist jedoch eine geringfügige Schräge 21 auf, derart, dass sich die Schräge von der dem Gewinde 19 abgewandten Seite zu der dem Gewinde 19 zugewandten Seite hin neigt.

Ferner weist die Klemmschraube 18 ein Drehmittel auf. Bei dem hier ausgeführten Ausführungsbeispiel ist das Drehmittel ein Inbus-Körper, der auf einfache Art und Weise in den Schraubenkopf eingeführt werden kann. Es entsteht nach dem Einführen ein Kraft- und Formschluss, sodass die entsprechende Drehbewegung beziehungsweise das entsprechende Drehmoment aufgebracht werden kann.

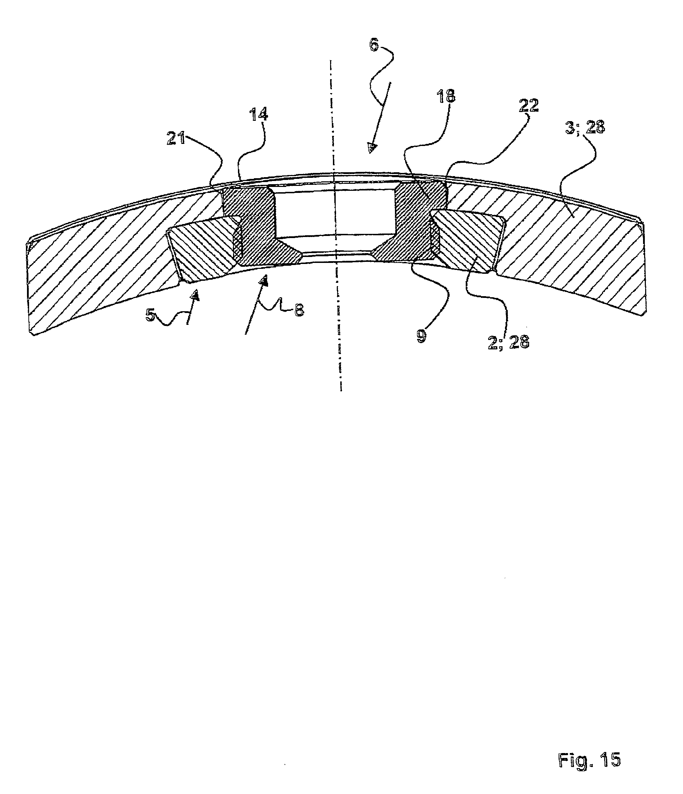

Fig. 15 zeigt einen Querschnitt durch ein Plattenimplantat 1. Der Querschnitt ist stellvertretend für alle Ausführungen des erfindungsgemässen Plattenimplantats 1 und zwar sowohl für die Grundausstattung, die aus einer ersten und zweiten Plattenkomponente 2, 3 besteht als auch für die Ausführungsbeispiele, die eine oder mehrere Erweiterungsplatten 28 aufweisen. Die Verbindung unter den einzelnen Plattenelementen ist immer die gleiche. Sie besteht in der Regel aus einem Verbindungsmittel 5, das in in einem Aufnahmemittel 6 geführt ist. Vorzugsweise ist seitlich ein entsprechendes Spiel vorgesehen, sodass eine leichte Führung des Verbindungsmittels 5 innerhalb des Aufnahmemittels 6 möglich ist. Der Querschnitt gemäss Fig. 15 zeigt nun den Grundkörper entweder der zweiten Plattenkomponente 3 oder einer Erweiterungsplatte 28. In diesem Grundkörper ist bereits das Verbindungsmittel 5 eingeschoben und derart weit geführt, dass die Bohrungen 8 des Verbindungsmittels 5 mit der Längsbohrung 14 des Grundkörpers fluchtend ist.

Ferner ist bereits die Klemmschraube 18 in den Grundkörper eingeführt. Die Klemmschraube 18 ist vollständig mit seinem Gewinde 19 in die Bohrung 8, das ein entsprechendes Gewinde 9 aufweist, eingedreht. Die Seitenflächen 21 der Klemmschraube 18 liegen bereits an den Seitenflanken 22 der Längsbohrung 14 an.

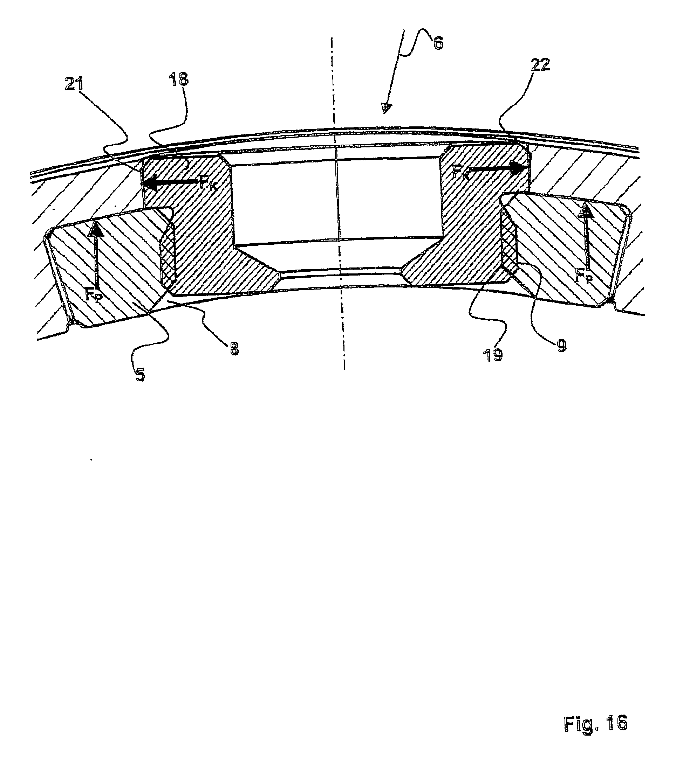

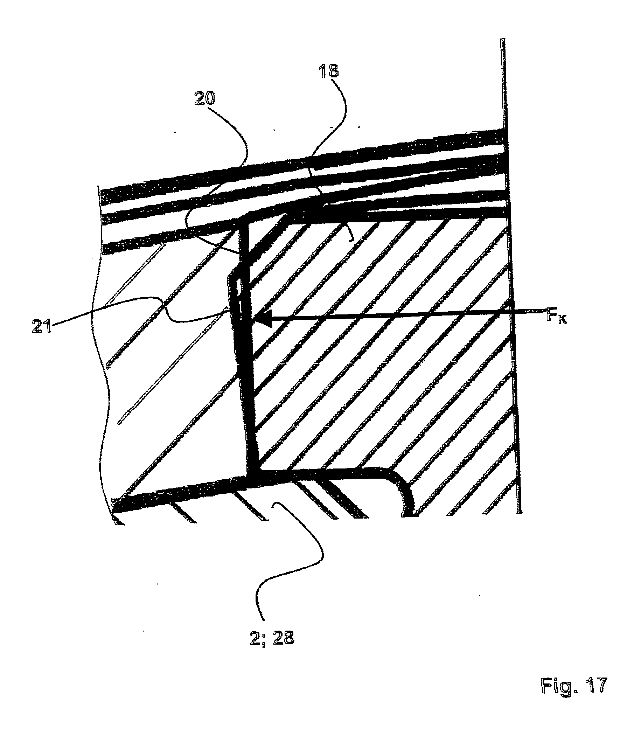

In Fig. 16 ist eine vergrösserte Darstellung der Fig. 15 gezeigt. Gegenüber Fig. 15 sind zusätzlich die Klemmkräfte eingezeichnet, die bei eingeschraubter Klemmschraube 18 wirken. Die erste Klemmkraft, die auftritt, ist diejenige, die dadurch hervorgerufen wird, dass die Klemmschraube eingedreht wird und in Verbindung mit dem Verbindungsmittel 5 steht. Durch das Einschrauben wird bewirkt, dass das Verbindungsmittel 5 unmittelbar an die Innenseite des Aufnahmemittels 6 gedrückt wird, wodurch Klemmkräfte F_{P} entstehen. Eine weitere Klemmkraft entsteht dadurch, dass die Schräge 21 der Klemmschraube 18 mit der Seitenflanke 22 der Längsbohrung 14 zusammenwirkt. Wie insbesondere aus Fig. 17 zu erkennen ist, steht die Seitenflanke auf der dem Gewinde 19 abgewandten Seite leicht in der Flucht über, sodass schon aufgrund dessen eine Klemmbeziehungsweise Keilwirkung entsteht. Dabei treten Klemmkräfte F_{K} auf. Bei gleichmässiger, symmetrischer Ausbildung der Längsbohrung sind die Klemmkräfte F_{K} nahezu gleich. Ist die Längsbohrung 14 jedoch konisch ausgestaltet, so nehmen die Klemmkräfte mit zunehmendem Erreichen des Anschlages (Wegbegrenzung der Gleitbewegung) zu.

Aufgrund der auftretenden Klemmkräfte F_{K}, F_{P} entsteht somit eine doppelte Sicherheit. Die auftretenden Klemmkräfte sind zudem in unterschiedliche Richtungen ausgerichtet, so dass diese sich nicht gegenseitig kompensieren können.

Durch geeignete Materialauswahl und der entsprechenden Auswahl der Oberflächen kann erreicht werden, dass keine Blockierung der möglichen Gleitbewegung erfolgt, sondern dass ständig gegen die ausgewählte Klemmkraft ein Gleiten des Verbindungsmittels innerhalb des Aufnahmemittels möglich ist.

Aufgrund der erfindungsgemässen Ausgestaltung des Plattenimplantats, das mindestens aus einer ersten Plattenkomponente und einer zweiten Plattenkomponente vorzugsweise aber aus zwischen der ersten Plattenkomponente und der zweiten Plattenkomponente dazwischen geschalteten Erweiterungsplatten besteht, ist ein Plattenimplantat geschaffen worden, das universell bei der Anwendung der Osteosynthese einsetzbar ist. Dieses Plattenimplantat zeichnet sich insbesondere dadurch aus, dass es das so genannte Settling, das bei der Rückbildung von versteiften Knochenelementen eintritt, ausgleicht, sodass der Kraftfluss, der normalerweise von den Knochen aufgenommen wird, auch über die Knochen weiterhin übertragen wird, sodass eine funktionelle Überbelastung des Plattenimplantats vermieden wird.

Durch die einfache Ausgestaltung und das Baukastenprinzip ist es möglich, Plattenimplantate unterschiedlicher Länge und Grösse auszuwählen. Durch einfaches Ineinanderfügen ist ein Vorfixieren ohne Probleme und ohne grösseren Aufwand möglich, sodass für den Anwender ein Anpassen, insbesondere im Bereich des Halswirbels auf sehr einfache Art und Weise vonstatten gehen kann.

### Bezugszeichenliste

- 1 :: Plattenimplantat
- 2 :: erste Plattenkomponente
- 3 :: zweite Plattenkomponente
- 4 :: Bohrung
- 5 :: Verbindungsmittel
- 6 :: Aufnahmemittel
- 7 :: freies Ende des Verbindungsmittels 5
- 8 :: Bohrung
- 9 :: Innengewinde
- 10 :: Quererstreckung
- 11 :: Längserstreckung
- 12 :: Aussparung
- 13 :: Pfeilrichtung
- 14 :: Längsbohrung
- 15 :: Bohrung
- 16 :: Bohrung
- 17 :: Befestigungsschraube
- 18 :: Klemmschraube
- 19 :: Gewinde (der Klemmschraube)
- 20 :: Wulst
- 21 :: Schräge
- 22 :: Seitenflanke
- 23 :: Pfeilrichtung
- 24 :: Abstand
- 25 :: Abstand
- 26 :: Wirbelsäule
- 27 :: Wirbelkörper
- 28 :: Erweiterungsplatte
- 29 :: Bohrung

## Patentansprüche

1. Plattenimplantat zur Anwendung bei einer Osteosynthese, bestehend aus mindestens einer ersten Plattenkomponente (2), die Bohrungen (4) zur Aufnahme von Befestigungsschrauben (17) und ein Verbindungsmittel (5) aufweist, mindestens einer zweiten Plattenkomponente (3), die Bohrungen (4) zur Aufnahme von Befestigungsschrauben (17) und ein Aufnahmemittel (6) zur Aufnahme des Verbindungsmittels (5) aufweist, wobei jede Plattenkomponente (2, 3) bezüglich der anderen Plattenkomponente (2, 3) in einer Richtung (23) gleiten kann und wobei die Plattenkomponenten (2, 3) mit einer Vorrichtung versehen sind, die ihren Gleitlauf zueinander begrenzt und eine Klemmschraube (18) und eine Längsbohrung (14), die in der zweiten Plattenkomponente (3) vorgesehen ist, umfasst, wobei die Klemmschraube (18) aus einem Schraubengewinde (19) und einen Schraubenkopf besteht und im montierten Zustand der beiden Plattenkomponenten (2, 3) das Schraubengewinde (19) mit dem Verbindungsmittel (5) und der Schraubenkopf mit der Längsbohrung (14) zusammenwirkt, derart, dass zwischen dem Schraubenkopf und der Längsbohrung (14) eine erste Klemmwirkung entsteht und zwischen dem Verbindungsmittel (5) und dem Aufnahmemittel (6) eine zweite Klemmwirkung entsteht, wobei die beiden Plattenkomponenten (2, 3) und die Klemmschraube (18) derart ausgebildet sind, dass in den Grenzen des Gleitlaufes kein Blockieren der Gleitbewegung in oder gegen die Richtung (23) erfolgt, und dass der Schraubenkopf der Klemmschraube (18) einen Wulst (20) aufweist, der mit den Seitenflanken (22) der Längsbohrung (14) zusammenwirkt, derart, dass die besagte erste Klemmwirkung entsteht und dadurch eine Klemmkraft zwischen der Klemmschraube (18) und der zweiten Plattenkomponente (3) erzeugt wird.

2. Plattenimplantat nach Anspruch 1,
**dadurch gekennzeichnet, dass**
zwischen der ersten Plattenkomponente (2) und der zweiten Plattenkomponente (2, 3) mindestens eine Erweiterungsplatte (28) vorgesehen ist, wobei die Erweiterungsplatte (28) Bohrungen für die Aufnahme von Befestigungsschrauben (29), ein Aufnahmemittel (6) zur Aufnahme eines Verbindungsmittels (5), ein Verbindungsmittel (5) und eine Längsbohrung (14) zur Aufnahme einer weiteren Klemmschraube (18) und zur Begrenzung des Gleitlaufs aufweist.

3. Plattenimplantat nach Anspruch 2,
**dadurch gekennzeichnet, dass** die Plattenkomponenten (2, 3) und die Erweiterungsplatte (28) aus Kunststoff sind.

4. Plattenimplantat nach Anspruch 2,
**dadurch gekennzeichnet, dass** die Plattenkomponenten (2, 3) und die Erweiterungsplatte (28) sowohl in ihrer Längserstreckung (11) als auch in der Quererstreckung (10) eine Krümmung aufweisen.

5. Plattenimplantat nach Anspruch 2,
**dadurch gekennzeichnet, dass** die Plattenkomponenten (2, 3) und/oder die Erweiterungsplatte (28) zusätzliche Bohrungen (16) zum Fixieren des Plattenimplantats (1) aufweist.

6. Plattenimplantat nach Anspruch 1,
**dadurch gekennzeichnet, dass** das Verbindungsmittel (5) zungenartig ist und auf der zum Knochen hinweisenden Seite nicht fluchtend mit der Unterseite der Plattenkomponenten (2, 3) ausgelegt ist.

7. Plattenimplantat nach einem der vorangegangenen Ansprüche, **dadurch gekennzeichnet, dass** der Wulst (20) eine Schräge (21) aufweist, derart, dass sich die Schräge (21) von der dem Gewinde (19) abgewandten Seite zu der dem Gewinde (19) zugewandten Seite nach innen hin neigt.

## Claims

1. A plate implant for use in osteosynthesis comprising at least one first plate component (2) having bores (4) for receiving fixing screws (17) and a connecting means (5), at least one second plate component (3) having bores (4) for receiving fixing screws (17) and a receiving means (6) for receiving the connecting means (5), wherein each plate component (2, 3) can slide with respect to the other plate component (2, 3) in a direction (23) and wherein the plate components (2, 3) are provided with a device which limits their sliding movement relative to each other and includes a clamping screw (18) and a longitudinal bore (14) which is provided in the second plate component (3), wherein the clamping screw (18) comprises a screw thread (19) and a screw head and in the assembled condition of the two plate components (2, 3) the screw thread (19) cooperates with the connecting means (5) and the screw head with the longitudinal bore (14) in such a way that a first clamping action is produced between the screw head and the longitudinal bore (14) and a second clamping action is produced between the connecting means (5) and the receiving means (6), wherein the two plate components (2, 3) and the clamping screw (18) are so designed that in the limits of the sliding movement no blocking of the sliding movement is effected in or in opposite relationship to the direction (23) and the screw head of the clamping screw (18) has a bead (20) which cooperates with the side flanks (22) of the longitudinal bore (14) in such a way that said first clamping action is produced and thereby a clamping force is produced between the clamping screw (18) and the second plate component (3).

2. A plate implant according to claim 1 **characterised in that** at least one extension plate (28) is provided between the first plate component (2) and the second plate component (2, 3), wherein the extension plate (28) has bores for receiving fixing screws (29), a receiving means (6) for receiving a connecting means (5), a connecting means (5) and a longitudinal bore (14) for receiving a further clamping screw (18) and for limiting the sliding movement.

3. A plate implant according to claim 2 **characterised in that** the plate components (2, 3) and the extension plate (28) are of plastic.

4. A plate implant according to claim 2 **characterised in that** the plate components (2, 3) and the extension plate (28) have a curvature both in their longitudinal extent (11) and also in the transverse extent (10).

5. A plate implant according to claim 2 **characterised in that** the plate components (2, 3) and/or the extension plate (28) has additional bores (16) for fixing the plate implant (1).

6. A plate implant according to claim 1 **characterised in that** the connecting means (5) is tongue-like and on the side facing towards the bone is designed in non-aligned relationship with the underside of the plate components (2, 3).

7. A plate implant according to one of the preceding claims **characterised in that** the bead (20) has an inclined portion (21) such that the inclined portion (21) is inclined inwardly from the side remote from the thread (19) to the side facing towards the thread (19).

## Revendications

1. Implant en forme de plaque destiné à être utilisé lors d'une ostéosynthèse, constitué d'au moins un premier composant de plaque (2) qui présente des trous (4), destinés à recevoir des vis de fixation (17), et un moyen d'assemblage (5), d'au moins un deuxième composant de plaque (3) qui présente des trous (4), destinés à recevoir des vis de fixation (17), et un moyen de réception (6) destiné à recevoir le moyen d'assemblage (5), chaque composant de plaque (2, 3) pouvant glisser dans une direction (23) par rapport à l'autre composant de plaque (2, 3), et les composants de plaque (2, 3) étant pourvus d'un dispositif qui limite leur course de glissement réciproque et comprend une vis de serrage (18) et un trou longitudinal (14) qui est prévu dans le deuxième composant de plaque (3), la vis de serrage (18) étant constituée d'un filetage (19) et d'une tête de vis et, à l'état monté des deux composants de plaque (2, 3), le filetage (19) coopérant avec le moyen d'assemblage (5) et la tête de vis avec le trou longitudinal (14), de telle sorte qu'un premier effet de serrage est créé entre la tête de vis et le trou longitudinal (14), et un deuxième effet de serrage est créé entre le moyen d'assemblage (5) et le moyen de réception (6), sachant que les deux composants de plaque (2, 3) et la vis de serrage (18) sont agencés de manière telle que dans les limites de la course de glissement il n'y ait pas de blocage du mouvement de glissement dans la direction (23) ou dans la direction opposée, et que la tête de vis de la vis de serrage (18) présente un bourrelet (20) qui coopère avec les flancs latéraux (22) du trou longitudinal (14), de manière à créer ledit premier effet de serrage et produire ainsi une force de serrage entre la vis de serrage (18) et le deuxième composant de plaque (3).

2. Implant en forme de plaque selon la revendication 1, **caractérisé en ce qu'**il est prévu, entre le premier composant de plaque (2) et le deuxième composant de plaque (2, 3), au moins une plaque d'extension (28), sachant que la plaque d'extension (28) présente des trous destinés à recevoir des vis de fixation (29), un moyen de réception (6) destiné à recevoir un moyen d'assemblage (5), un moyen d'assemblage (5) et un trou longitudinal (14) destiné à recevoir une autre vis de serrage (18) et à limiter la course de glissement.

3. Implant en forme de plaque selon la revendication 2, **caractérisé en ce que** le composant de plaque (2, 3) et la plaque d'extension (28) sont réalisés en matière synthétique.

4. Implant en forme de plaque selon la revendication 2, **caractérisé en ce que** les composants de plaque (2, 3) et la plaque d'extension (28) présentent une courbure aussi bien dans le sens de leur dimension longitudinale (11) que dans le sens de leur dimension transversale (10).

5. Implant en forme de plaque selon la revendication 2, **caractérisé en ce que** les composants de plaque (2, 3) et/ou la plaque d'extension (28) présente(nt) des trous (16) supplémentaires, destinés à la fixation de l'implant en forme de plaque (1).

6. Implant en forme de plaque selon la revendication 1, **caractérisé en ce que** le moyen d'assemblage (5) se présente sous forme de languette et est conçu, sur le côté tourné vers l'os, de manière à ne pas être aligné avec la face inférieure des composants de plaque (2, 3).

7. Implant en forme de plaque selon l'une des revendications précédentes, **caractérisé en ce que** le bourrelet (20) présente un chanfrein (21), de telle sorte que le chanfrein (21) soit incliné vers l'intérieur depuis le côté éloigné du filetage (19) vers le côté tourné vers le filetage (19).
